# FASCICULE DE BREVET EUROPEEN

(11) **EP 0 722 717 B1**
(45) Date de publication et mention de la délivrance du brevet: **19.03.1997**
(21) Numéro de dépôt: 96400008.7
(22) Date de dépôt: 02.01.1996
(51) Int. Cl.: A61K 7/48, A23L 1/05, C09K 3/00, C10M 115/08, C09D 7/12

(54) **Utilisation de céramides en tant qu'agent épaississant**
Verwendung von Ceramiden als Verdickungsmittel
Use of ceramids as thickening agents

(30) Priorité: 20.01.1995 FR 9500660
(43) Date de publication de la demande: 24.07.1996
(73) Titulaire: L'OREAL, 75008 Paris (FR)
(72) Inventeur: Philippe, Michel, F-91320 Wissous (FR); Mahieu, Claude, F-75017 Paris (FR); Semeria, Didier, F-77181 Courtry (FR); Ribier, Alain, F-75001 Paris (FR)
(74) Mandataire: Tezier Herman, Béatrice

(56) Documents cités:
- EP-A- 0 556 957
- GB-A- 2 231 871

## Description

L'invention se rapporte à l'utilisation de céramides naturels ou synthétiques en tant qu'agent épaississant de milieux en particulier non aqueux.

Les agents épaississants de milieux en particulier non aqueux couramment utilisés sont essentiellement des N-acylaminoacides comme ceux décrits dans la demande de brevet FR 2 281 162, des sels d'acides gras, tels ceux d'aluminium, de magnésium ou de calcium comme ceux décrits le brevet US 2789329, des esters glycérolés d'acides gras, des esters gras de dextrine, des colloïdes inorganiques tels que la bentone, ou bien encore des produits naturels comme la cire d'abeille ou la cire de carnauba.

Les dérivés d'acides aminés et les différents sels d'acides gras sont des molécules ioniques. Il en découle qu'ils sont difficilement compatibles avec l'ensemble des différents adjuvants que l'on utilise dans les compositions. Ceci a pour conséquence d'entraîner des difficultés de formulation.

De plus si l'on utilise des esters, ceux-ci sont généralement sous la forme de mélange d'esters dont les proportions sont variables et souvent peu définies. Les esters présentent également une grande sensibilité au pH. On retrouve là encore des difficultés de formulation.

On peut également citer les dérivés non ioniques de synthèse, de la famille des acides 11-N-alkyloxycarbonylamino-undécanoïques et leurs esters, décrits dans la demande de brevet FR 2 647 445 (GB-A-2 231 871). Ces composés présentent le désavantage de ne pas permettre l'obtention de solutions transparentes. De plus on constate une certaine instabilité entraînant l'apparition de cristaux dans les solutions épaissies les contenant.

Les céramides, qui à l'état naturel sont les composants principaux des couches lipidiques de l'épiderme, sont utilisés en cosmétique sous forme naturelle ou synthétique dans des compositions destinées à renforcer l'effet barrière du stratum cornéum afin de réduire la perte en eau et donc le dessèchement de la peau (GB 2 178 312, GB 2 213 723, EP 227 994, EP 282 616, EP 556 957).

Ils sont également utilisés dans des compositions cosmétiques pour leurs propriétés conférants à la peau une meilleure élasticité (EP 500 437).

C'est de manière surprenante et inattendue que la demanderesse a pu montrer que des céramides naturels ou synthétiques peuvent être utilisés en tant qu'agent épaississant d'un milieu.

L'utilisation de ces composés permet d'obtenir un milieu épaissi ne présentant pas les désavantages connus conférés par les produits de l'art antérieur.

De plus, dans la mesure où il s'agit de composés habituellement présents dans la peau, ils présentent l'avantage de ne pas être irritants pour celle-ci. Leur utilisation en cosmétique apparaît donc comme avantageuse.

L'invention a donc pour objet l'utilisation dans un milieu, en tant qu'agent épaississant, de composés répondant à la formule (I) suivante:

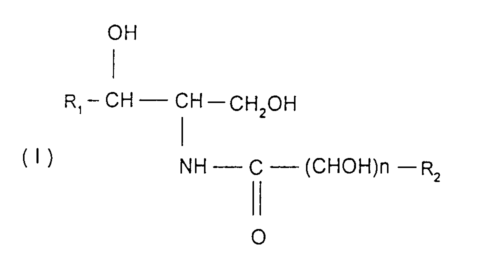

Formule (I) dans laquelle R₁ représente un radical hydrocarboné linéaire ou ramifié, saturé ou insaturé, ayant de 9 à 25 atomes de carbone,
R₂ représente un radical hydrocarboné linéaire ou ramifié, saturé ou insaturé, ayant de 5 à 29 atomes de carbone et
n prend les valeurs 0 ou 1,
ou d'au moins l'un de ses isomères optiques ou l'un des diastéréoisomères,
R₁ contient de préférence entre 11 et 23 atomes de carbone.
R₂ contient de préférence entre 7 et 25 atomes de carbone.

L'agent épaississant peut être un mélange de composés de formule (I).

Ces composés peuvent être utilisés sous forme d'isomère pur ou de mélange d'isomères.

On préfère utiliser comme composés de formule (I) :
- le 2-octanoylamino-octadécane-1,3-diol,
- le 2-hexadécanoylamino-octadécane-1,3-diol,
- le 2-oléoylamino-octadécane-1,3-diol,
- le 2-tetradécanoylamino-octadécane-1,3-diol.

On peut plus particulièrement utiliser ces agents épaississants dans les domaines de la cosmétique, de la pharmacie, des peintures et vernis, des lubrifiants, des combustibles et de l'industrie alimentaire.

Ces composés ont une propriété épaississante remarquable. Cette propriété a pour conséquence de permettre l'utilisation de faibles quantités de ce type de composé dans les milieux à épaissir.

Ces composés permettent d'obtenir des milieux épaissis qui présentent une homogénéité stable dans le temps.

La quantité de composés de formule (I) dans le milieu à épaissir dépend, bien entendu, du degré d'épaississement que l'on désire obtenir. A titre indicatif, les composés de formule (I) peuvent être utilisés à des concentrations comprises entre 0,1 % et 20 %, et de préférence entre 1 % et 10 % en poids par rapport au poids total du milieu.

Le tableau ci-dessous donne un exemple non limitatif des proportions de 2-oléoylamino-octadécane-1,3-diol nécessaires pour épaissir différents milieux non aqueux:

| | |
|---|---|
| huile de vaseline (Marcol 82 de chez Esso) | 2 % |
| huile de colza (Huilerie de Lapalisse) | 1 % |
| miglyol 812^{TM} (Dynamit Nobel) | 1 % |
| Finsolv_{TN} (Witco) | 2 % |
| cyclométhicone (cyclopenta diméthylesiloxane de Union Carbide) | 2 % |
| 1-(2'-f-hexyléthylthio)-3-(2''-éthylhexyloxy)-2-propanol (FR 2684668) | 2 % |
| Eutanol G (Henkel) | 5 % |
| toluène | 2 % |
| D.M.S.O.^{*} | 5 % |
| myristate d'isopropyle | 2 % |

| | |
|---|---|
| ^{*} : dimethylsulfoxyde | |

Le milieu peut donc être aqueux et de préférence non aqueux.

Lorsqu'il est aqueux il peut notamment être constitué d'eau, d'un mélange d'eau et de liquide organique ou encore d'un mélange hydroalcoolique.

Le milieu non aqueux est constitué d'au moins un liquide organique, tel que, par exemple, un dérivé hydrocarboné saturé ou insaturé, comme par exemple l'huile de vaseline, le perhydrosqualène, l'huile de colza, l'huile de jojoba ; un ester gras aliphatique ou aromatique tel que les alkyles benzoates, l'octyle stéarate, le myristate d'isopropyle un triglycéride tel que les triglycérides d'acide caprique et/ou caprylique, (miglyol 812™) ; un polydiméthylsiloxane tel que le cyclométhicone ; un alcool gras tel que l'octyldodécanol (Eutanol G) ; ou un solvant tel que le toluène, le diméthylsulfoxyde.

Il est bien entendu que le milieu non aqueux peut être constitué par le mélange de 2 ou plusieurs des éléments cités ci-dessus, pris seuls ou en combinaison avec d'autres composants non aqueux qui n'auraient pas été cités.

L'épaississement du milieu peut s'effectuer par solubilisation complète du composé de formule I dans le milieu à épaissir, à une température comprise entre la température ambiante et la température d'ébullition du liquide constituant le milieu, puis en laissant reposer le mélange obtenu jusqu'à ce qu'il soit totalement épaissi. De préférence, le liquide est un liquide organique.

Le milieu ainsi épaissi peut constituer ou être un constituant d'une composition cosmétique ou pharmaceutique, de peintures ou de vernis, de lubrifiants, de combustibles ou encore de produits alimentaires.

Ces compositions sont préférentiellement non aqueuses.

En cosmétique ou en pharmacie, une telle composition peut, entre autre, se présenter sous forme de sticks, de laits ou de vernis.

Elle comprend, alors, les ingrédients habituellement utilisés en cosmétique ou en pharmacie. Ainsi, elle peut comprendre notamment au moins un additif choisi parmi les alcools gras, les épaississants, les esters d'acides gras, les esters d'acides gras et de glycérol, les silicones (volatiles ou non, fonctionnalisés ou non), les tensioactifs, les parfums, les conservateurs, les filtres solaires, les protéines, les vitamines, les polymères, les huiles organiques ou inorganiques.

Toutes ces compositions sont préparées selon les méthodes usuelles connues de l'homme de l'art.

### EXEMPLES

On va maintenant donner à titre d'illustration des exemples de divers milieux épaissis par des composés répondant à la formule (I), ainsi que des exemples de compositions les comprenant. Ces exemples ne sauraient limiter en aucune façon la portée de l'invention.

### Exemple 1 : Gel anhydre pour peaux très sèches :

| | |
|---|---|
| 2-oléoylamino-octadécane-1,3-diol | 5,0 % |
| palminate de vitamine A* | 0,2 % |
| acétate de tocophérol | 20,0 % |
| huile de vaseline | 10,0 % |
| Eutanol G | 34,8 % |
| cyclométhicone | 30,0 % |

| | |
|---|---|
| ^{*} : à 10⁶ U.I./g dans l'huile de cacahuète | |

La composition obtenue se présente sous l'aspect d'un gel qui ne coule pas, facilement préhensible au doigt. Cette composition s'étale aisément sur la peau.

### Exemple 2 : fluide épais anti-age :

| | |
|---|---|
| 2-oléoylamino-octadécane-1,3-diol | 2,0 % |
| palminate de vitamine A^{*} | 0,5 % |
| acétate de tocophérol | 25,5 % |
| huile de vaseline | 12,0 % |
| Eutanol G | 30,0 % |
| cyclométhicone | 30,0 % |

| | |
|---|---|
| ^{*} : à 10⁶ U.I./g dans l'huile de cacahuète | |

Cette composition se présente sous l'aspect d'une crème fluide de consistance moyennement épaisse, qui s'étale et pénètre aisément la peau.

### Exemple 3 : Shampooing crème traitant :

| | |
|---|---|
| 2-oléoylamino-octadécane-1,3-diol | 3,0 % |
| Alkyl-polyglucoside | 10,0 % |
| Conservateur | 0,1 % |
| eau | qsp 100,0 % |

Ce shampooing se présente sous la forme d'une crème épaisse qui s'applique directement sur les cheveux. Il procure une mousse volumineuse et confère à la chevelure un toucher agréable.

## Revendications

1. Utilisation en tant qu'agent épaississant d'un milieu, d'au moins un composé de formule générale (I) : 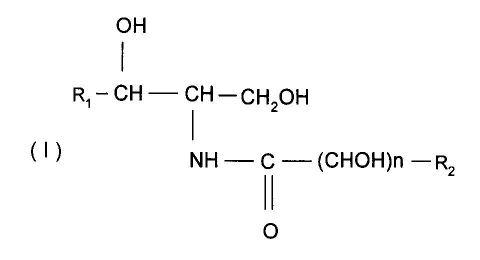 dans laquelle R₁ représente un radical hydrocarboné linéaire ou ramifié, saturé ou insaturé, ayant de 9 à 25 atomes de carbone,
R₂ représente un radical hydrocarboné linéaire ou ramifié, saturé ou insaturé, ayant de 5 à 29 atomes de carbone et
n prend les valeurs 0 ou 1,
ou d'au moins l'un de ses isomères optique ou l'un des diastéréoisomères.

2. Utilisation selon la revendication 1, dans laquelle le milieu est un milieu non aqueux.

3. Utilisation selon l'une quelconque des revendications 1 ou 2, pour laquelle R₁ est un radical hydrocarboné ayant de 11 à 23 atomes de carbone.

4. Utilisation selon l'une quelconque des revendications 1 à 3, pour laquelle R₂ est un radical hydrocarboné ayant de 7 à 25 atomes de carbone.

5. Utilisation selon l'une quelconque des revendications 1 à 4, pour laquelle la quantité de composé de formule I est comprise entre 0,1 % et 20 % en poids par rapport au poids total du milieu.

6. Utilisation selon l'une quelconque des revendications 1 à 5, pour laquelle la quantité de composé de formule (I) est comprise entre 1 % et 10 % en poids par rapport au poids total du milieu.

7. Utilisation selon l'une quelconque des revendications 1 à 6, pour laquelle le composé de formule (I) est choisi parmi:
- le 2-octanoylamino-octadécane-1,3-diol,
- le 2-hexadécanoylamino-octadécane-1,3-diol,
- le 2-oléoylamino-octadécane-1,3-diol,
- le 2-tetradécanoylamino-octadécane-1,3-diol.

8. Utilisation selon l'une quelconque des revendications 2 à 7, dans laquelle le milieu non aqueux est constitué d'au moins un dérivé hydrocarboné saturé ou insaturé, un ester gras aliphatique ou aromatique, un triglycéride, un polydiméthylsiloxane, un alcool, ou un solvant.

9. Utilisation selon la revendication 8, dans laquelle le milieu non aqueux est choisi parmi l'huile de vaseline, le perhydrosqualène, l'huile de colza, l'huile de colza, l'huile de jojoba les esters gras aliphatiques ou aromatiques tels que les alkyles benzoates, l'octyle stéarate, le myristate d'isopropyle, les triglycérides tels que les triglycérides d'acide caprique et/ou caprylique (miglyol 812™), les polydiméthylsiloxanes tels que le cyclométhicone les alcools gras tels que l'octyldodécanol (Eutanol G), les solvants tels que le toluène, le dimethylsulfoxyde.

10. Utilisation du milieu épaissi tel que défini dans l'une quelconque des revendications 1 à 9, dans des compositions cosmétiques ou pharmaceutiques, des peintures ou des vernis, des lubrifiants, des combustibles ou encore des produits alimentaires.

11. Utilisation selon la revendication précédente, dans des compositions cosmétiques ou pharmaceutiques.

## Claims

1. Use, as an agent for thickening a medium, of at least one compound of general formula (I): 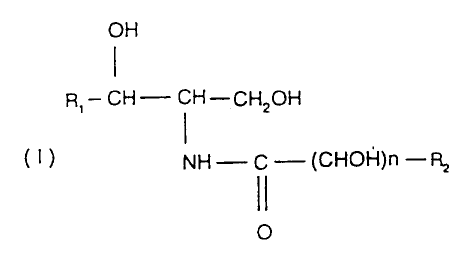 in which R₁ represents a saturated or unsaturated linear or branched hydrocarbon radical having from 9 to 25 carbon atoms,
R₂ represents a saturated or unsaturated linear or branched hydrocarbon radical having from 5 to 29 carbon atoms, and
n takes the values 0 or 1,
or at least one of the optical isomers thereof or one of the diastereoisomers.

2. Use according to Claim 1, in which the medium is a non-aqueous medium.

3. Use according to either of Claims 1 and 2, for which R₁ is a hydrocarbon radical having from 11 to 23 carbon atoms.

4. Use according to any one of Claims 1 to 3, for which R₂ is a hydrocarbon radical having from 7 to 25 carbon atoms.

5. Use according to any one of Claims 1 to 4, for which the amount of compound of formula (I) is between 0.1% and 20% by weight relative to the total weight of the medium.

6. Use according to any one of Claims 1 to 5, for which the amount of compound of formula (I) is between 1% and 10% by weight relative to the total weight of the medium.

7. Use according to any one of Claims 1 to 6, for which the compound of formula (I) is chosen from:
- 2-octanoylaminooctadecane-1,3-diol,
- 2-hexadecanoylaminooctadecane-1,3-diol,
- 2-oleoylaminooctadecane-1,3-diol,
- 2-tetradecanoylaminooctadecane-1,3-diol.

8. Use according to any one of Claims 2 to 7, in which the non-aqueous medium consists of at least one saturated or unsaturated hydrocarbon derivative, an aliphatic or aromatic fatty ester, a triglyceride, a polydimethylsiloxane, an alcohol or a solvent.

9. Use according to Claim 8, in which the non-aqueous medium is chosen from liquid petrolatum, perhydrosqualene, rapeseed oil, jojoba oil, aliphatic or aromatic fatty esters such as alkyl benzoates, octyl stearate, isopropyl myristate, triglycerides such as capric and/or caprylic acid triglycerides (miglyol 812™), polydimethylsiloxanes such as cyclomethicone, fatty alcohols such as octyldodecanol (Eutanol G), and solvents such as toluene and dimethyl sulphoxide.

10. Use of the thickened medium as defined in any one of Claims 1 to 9, in cosmetic or pharmaceutical compositions, paints or varnishes, lubricants, combustibles or alternatively food products.

11. Use according to the preceding claim, in cosmetic or pharmaceutical compositions.

## Patentansprüche

1. Verwendung mindestens einer Verbindung der allgemeinen Formel (I) 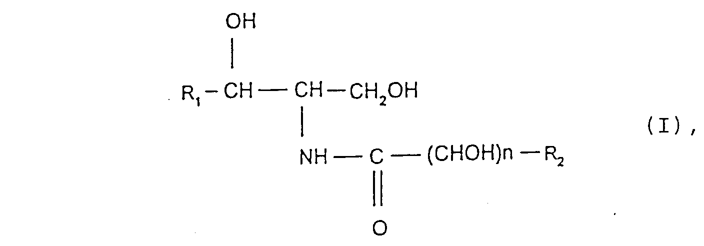 in der bedeuten:
- R₁ einen geradkettigen oder verzweigten, gesättigten oder ungesättigten Kohlenwasserstoffrest mit 9 bis 25 Kohlenstoffatomen,
- R₂ einen geradkettigen oder verzweigten, gesättigten oder ungesättigten Kohlenwasserstoffrest mit 5 bis 29 Kohlenstoffatomen und
- n eine Zahl, deren Wert 0 oder 1 beträgt,
oder mindestens eines ihrer optischen Isomeren oder eines der Diastereomeren als Verdickungsmittel eines Mediums.

2. Verwendung nach Anspruch 1, wobei das Medium ein nicht-wäßriges Medium ist.

3. Verwendung nach einem der Ansprüche 1 oder 2, wobei R₁ ein Kohlenwasserstoffrest mit 11 bis 23 Kohlenstoffatomen ist.

4. Verwendung nach einem der Ansprüche 1 bis 3, wobei R₂ ein Kohlenwasserstoffrest mit 7 bis 25 Kohlenstoffatomen ist.

5. Verwendung nach einem der Ansprüche 1 bis 4, wobei die Menge der Verbindung der Formel (I) 0,1 bis 20 Gew.-%, bezogen auf das Gesamtgewicht des Mediums, beträgt.

6. Verwendung nach einem der Ansprüche 1 bis 5, wobei die Menge der Verbindung der Formel (I) 1 bis 10 Gew.-%, bezogen auf das Gesamtgewicht des Mediums, beträgt.

7. Verwendung nach einem der Ansprüche 1 bis 6, wobei die Verbindung der Formel (I) ausgewählt wird unter
- 2-Octanoylaminooctadecan-1,3-diol,
- 2-Hexadecanoylaminooctadecan-1,3-diol,
- 2-Oleoylaminooctadecan-1,3-diol,
- 2-Tetradecanoylaminooctadecan-1,3-diol.

8. Verwendung nach einem der Ansprüche 2 bis 7, wobei das nichtwäßrige Medium aus mindestens einem gesättigten oder ungesättigten Kohlenwasserstoffderivat, aliphatischen oder aromatischen Fettester, Triglycerid, Polydimethylsiloxan, Alkohol oder Lösungsmittel besteht.

9. Verwendung nach Anspruch 8, wobei das nichtwäßrige Medium unter Vaselineöl, Perhydrosqualen, Rapsöl, Jojobaöl, aliphatischen oder aromatischen Fettestern, wie z.B. Alkybenzoaten, Octylstearat und Isopropylmyristat, Triglyceriden, wie z.B. Triglyceriden von Caprin- und/oder Caprylsäure (Miglyol® 812), Polydimethylsiloxanen, wie z.B. Cyclomethicon, Fettalkoholen, wie z.B. Octyldodecanol (Eutanol G) und Lösungsmitteln, wie z.B. Toluol oder Dimethylsulfoxid, ausgewählt wird.

10. Verwendung des in einem der Ansprüche 1 bis 9 definierten verdickten Mediums in kosmetischen oder pharmazeutischen Zusammensetzungen, Farben und Lacken, Schmiermitteln, Brennstoffen und Lebensmitteln.

11. Verwendung nach dem vorhergehenden Anspruch in kosmetischen oder pharmazeutischen Zusammensetzungen.
